# EUROPÄISCHE PATENTSCHRIFT

(11) **EP 4 025 261 B1**
(45) Veröffentlichungstag und Bekanntmachung des Hinweises auf die Patenterteilung: **07.06.2023**
(21) Anmeldenummer: 21805360.1
(22) Anmeldetag: 22.10.2021
(51) Int. Cl.: A61L 2/07

(54) **VORRICHTUNG UND VERFAHREN ZUM STERILISIEREN UND TROCKNEN VON MEDIZINISCHEN FILTERN**
DEVICE AND METHOD FOR STERILISING AND DRYING MEDICAL FILTERS
DISPOSITIF ET MÉTHODE DE STÉRILISATION ET DE SÉCHAGE DE FILTRES MÉDICAUX

(30) Priorität: 26.10.2020 DE 102020128083
(43) Veröffentlichungstag der Anmeldung: 13.07.2022
(73) Patentinhaber: Allmed Dialysis Technologies GmbH, 01896 Pulsnitz (DE)
(72) Erfinder: DRÖSCHEL, Stefan, 66687 Wadern (DE)
(74) Vertreter: Patentanwaltskanzlei Vièl & Wieske PartGmbB
(86) Internationale Anmeldenummer: PCT/DE2021/100851
(87) Internationale Veröffentlichungsnummer: WO 2022/089685

(56) Entgegenhaltungen:
- CN-A- 111 167 778
- DE-T5-112005 002 948
- US-A- 4 609 728
- US-A1- 2003 074 862
- US-B2- 9 555 146

## Beschreibung

Die Erfindung betrifft eine Vorrichtung zum Sterilisieren und Trocknen von medizinischen Filtern wie Dialysatoren, wobei die Vorrichtung eine Druckkammer mit mindestens einem Einlass und einem Auslass und eine Halterung für mindestens einen Filter aufweist, sowie ein Verfahren zum Sterilisieren und Trocknen von Filtern.

Zu den medizinischen Filtern gemäß der vorliegenden Erfindung zählen insbesondere Dialysatoren, insbesondere Hohlfaserdialysatoren, bei denen die vom Blut durchströmten Hohlfasern die Filtermembran bilden. Diese werden in der Hämodialyse eingesetzt. Sie bestehen aus mindestens einem Hohlfasermodul, durch welches von einer Bluteinlassöffnung Blut zu einer Blutauslassöffnung geleitet werden kann. Die Bluteinlassöffnung und die Blutauslassöffnung begrenzen dabei einen Blutraum. Die Dialysatoren besitzten zudem eine Einlassöffnung und eine Auslassöffnung für die Dialyseflüssigkeit. Die Dialyseflüssigkeit umspült die Hohlfasern, wobei die aus dem Blut zu filternden Stoffe durch die Hohlfasermebran vom Blut in die Dialyseflüssigkeit übergehen.

Durch den direkten Blutkontakt des Blutraums des Dialysators mit dem Blut des Patienten muss dieser steril sein.

Sterilisationsverfahren für Dialysatoren beruhen beispielsweise auf der Nutzung oder Verwendung von Gammastrahlung, Elektronenstrahlung, Ethylenoxid (EtO) und Heißdampf. In diesen Verfahren sind Strahlung, Hitze und Toxizität die auf Mikroorganismen abtötend wirkenden Effekte.

In der Medizintechnik werden zur Heißdampfsterilisation von medizinischen Artikeln Autoklaven eingesetzt. Dabei erfolgt die Sterilisation durch Verdrängen der Luft, Beaufschlagen von Heißdampf während einer bestimmten Zeit und einem nachfolgenden Trocknungsvorgang. Durch die Sterilisation werden gesundheitsgefährdende Keime und Mikroorganismen abgetötet. Bei der Sterilisation von Dialysatoren in Autoklaven hat sich als nachteilig herausgestellt, dass das Trocknen der hydrophilen Hohlfasern nach der Heißdampfsterilisation schwierig und zeitaufwendig ist.

Aus der AT 351 172 ist eine Sterilisierungsanlage für in Behälter abgefüllte Infusionslösungen oder dergleichen bekannt, deren Gehäuse eine Aufheizkammer, eine Sterilisierkammer und eine Abkühlkammer aufweist.

Die DE 11 2005 002 948 T5 beschreibt einen Wasserstoffperoxid-Dampfsterilisator und ein Sterilisationsverfahren zum Gebrauch desselben.

Die US 2003/0074862 A1 beschreibt ein Verfahren zur Dampfsterilisation von medizinischen Produkten, wobei die medizinischen Produkte separat oder zu mehreren in einen Karton gepackt werden, der versiegelt wird und dann die medizinischen Produkte in dem versiegelten Karton einer Dampfsterilisation unterzogen werden.

Die US 4,609,728 A beschreibt ein Verfahren zum Behandeln einer Zellulosehohlfaser, welche besonders für die Hämodialyse oder eine andere Blutbehandlung außerhalb des Körpers geeignet ist.

Aus der US 9,555,146 B2 ist ein Verfahren zum Sterilisieren eines Gegenstands, der mindestens eine erste und eine zweite Öffnung zu einem Innenraum des Gegenstands hin aufweist bekannt, umfassend das Verbinden der ersten Öffnung mit einer Zufuhrleitung für ein Sterilisationsmittel, das Verbinden der zweiten Öffnung mit einer Auslassleitung für das Sterilisationsmittel und das Leiten des Sterilisationsmittels durch die Zufuhrleitung in den Innenraum, durch den Innenraum und aus dem Innenraum durch die Auslassleitung hinaus.

Der Erfindung liegt die Aufgabe zugrunde, das Sterilisieren und Trocknen von Filtern, insbesondere von medizinischen Filtern, zu optimieren.

Die Aufgabe wird bei einer Vorrichtung gemäß dem Oberbegriff des Anspruchs 1 dadurch gelöst, dass die Druckkammer in eine erste Kammer und eine zweite Kammer unterteilt ist, wobei der Filter derart in die Halterung einlegbar ist, dass die Einlassöffnung(en) des Filters in der ersten Kammer und die Auslassöffnung(en) des Filters in der zweiten Kammer angeordnet sind.

Die erste und zweite Kammer kann dabei mit einem Druck zwischen 0 und 10 bar beaufschlagt werden.

Vorteilhaft kann der Filter, bei dem es sich um einen Dialysator handeln kann, in die Halterung der Vorrichtung eingelegt werden. Dabei sind die Bluteinlassöffnung und die Einlassöffnung für die Dialyseflüssigkeit in der ersten Kammer sowie die Blutauslassöffnung und die Auslassöffnung für die Dialyseflüssigkeit in der zweiten Kammer angeordnet. Entsprechendes gilt für andere Filter bzw. medizinische Filter. Durch den Einlass der Kammern kann sowohl die Restluft evakuiert werden als auch heißer Dampf oder Trockenluft zugegeben werden. Werden beide Kammern gleichzeitig betrieben, ist auch eine Sterilisation nach z. B. einem Vakuum - Dampf Verfahren möglich.

Vorteilhaft kann aber auch zur Dampfsterilisation in die erste Kammer durch den Einlass Heißdampf eingelassen werden. Dabei wird ein Überdruck in der ersten Kammer erzeugt. Es entsteht ein Druckgefälle zwischen der ersten Kammer und der zweiten Kammer. Dabei kann der Druckunterschied gering sein, um ein langsames Durchströmen des Filters mit dem Dampf zu ermöglichen. Beispielsweise kann der Druckunterschied 100 mbar bei einer Dampftemperatur von 123 °C sein. Durch den Druckunterschied zwischen den beiden Kammern durchströmt Heißdampf den Blut- und Dialysatraum des Dialysators von der Bluteinlassöffnung/Dialysateinlassöffnung zu der Blutauslassöffnung/Dialysatauslassöffnung, wodurch zunächst die vorliegende Luft verdrängt wird. Anschließend wird der Dialysator durch Beaufschlagen mit Heißdampf während einer festgelegten Zeit sterilisiert. Nach dem Austreten durch die Blutauslassöffnung/Dialysatauslassöffnung kann der Heißdampf aus der zweiten Kammer über den Auslass ausgeleitet werden. Entsprechendes gilt für andere Filter bzw. medizinische Filter. Es ist auch eine Kombination des Vakuumdampfverfahrens mit einer permanenten Dampfströmung durch den medizinischen Filter möglich. Der Temperaturbereich bei der Dampfsterilisation kann zwischen 100 - 180 °C liegen. Die Dauer der Dampfsterilisation kann beispielsweise zwischen 5 und 180 min liegen.

Das Trocken des Filters kann durch das mehrmalige, gleichzeitige evakuieren der beiden Kammern und anschließendes Befüllen mit trockener Luft erfolgen.

Vorteilhaft kann zum Trocknen des Dialysators nach dem Dampfsterilisieren warme Luft durch den Einlass in die erste Kammer eingeleitet werden. Es entsteht ein Druckgefälle zwischen der ersten Kammer und der zweiten Kammer. Dadurch durchströmt die warme Luft den Blut- und Dialysatraum des Dialysators, wodurch die Hohlfasern getrocknet werden. Die Feuchtigkeit wird dabei von der warmen Luft aufgenommen. Nach dem Austreten durch die Blutauslassöffnung/Dialysatauslassöffnung kann die warme Luft aus der zweiten Kammer über den Auslass ausgeleitet werden. Besonders vorteilhaft kann dadurch der Dialysator in weniger als einer halben Stunde, besonders vorteilhaft in 20 Minuten getrocknet werden. Entsprechendes gilt für andere Filter bzw. medizinische Filter. Die Vorrichtung kann dabei sowohl in horizontaler als auch vertikaler Ausrichtung betrieben werden.

Dabei ist es zweckmäßig, dass die Druckkammer durch eine Trennwand in eine erste Kammer und eine zweite Kammer unterteilt ist.

Dabei ist vorteilhaft vorgesehen, dass die Position der Trennwand verstellbar ist.

Durch die verstellbare Trennwand kann die Größe der ersten Kammer und der zweiten Kammer variiert werden.

Eine Ausgestaltung der Erfindung besteht darin, dass die erste Kammer größer als die zweite Kammer ist.

Besonders vorteilhaft kann dabei ein größerer Bereich des Dialysators in der ersten Kammer angeordnet sein. Dabei kann der Dialysator beim Einströmen des Dampfes auch von der Außenseite des Dialysators erhitzt werden, wodurch die Sterilisation begünstigt wird. Auch das Trocknen kann beschleunigt werden, indem warme Luft in die erste Kammer eingeströmt wird, die auch die Außenseite des Filters erwärmen kann und somit den Trocknungsprozess beschleunigen kann. Entsprechendes gilt für andere Filter bzw. medizinische Filter.

Eine weitere Ausgestaltung der Erfindung besteht darin, dass im Bereich der Halterung Mittel zum Aufbringen einer Kraft auf einen in der Halterung befindlichen Filter vorgesehen sind.

Insbesondere bei Dialysatoren mit Gehäusen aus Polypropylen dehnt sich dieses bei der Dampfsterilisation aus. Durch das Aufbringen einer Kraft auf die Dialysatoren während der Dampfsterilisation kann das Ausdehnen der Dialysatoren begrenzt werden. Dabei kann die Ausdehnung der Dialysatoren auf weniger als 5 mm, bevorzugt weniger als 2 mm, besonders bevorzugt auf weniger als 1 mm begrenzt werden. Vorteilhaft wird dadurch die Ondulierung der Hohlfaserbündel während der Dampfsterilisation erhalten. Der Kraftauftrag kann dabei abhängig von dem zu sterilisierenden Dialysator und der Ondulierung der Hohlfasern eingestellt werden.

Eine vorteilhafte Ausgestaltung der Erfindung besteht darin, dass zum Aufbringen der Kraft auf einen in der Halterung befindlichen Filter ein Federelement oder ein Druckzylinder vorgesehen ist.

Durch das Federelement bzw. den Druckzylinder kann die Kraft besonders einfach auf den Filter aufgebracht werden.

Eine weitere Ausgestaltung der Erfindung besteht darin, dass die Vorrichtung im Boden der ersten Kammer und der zweiten Kammer jeweils einen Ablauf für Kondenswasser aufweist.

Vorteilhaft kann über den Ablauf kondensierter Dampf aus der Druckkammer geleitet werden. Beispielsweise können Kondensatabscheider in beiden Kammern vorgesehen sein.

Eine weitere Ausgestaltung der Erfindung besteht darin, dass die erste Kammer und die zweite Kammer durch ein Ventil miteinander verbunden sind.

Dabei können beim Einleiten des Dampfes zur Dampfsterilisation die erste Kammer und die zweite Kammer über das offene Ventil verbunden sein. Der Dampf kann sich dadurch in der gesamten Druckkammer verteilen und durch alle Öffnungen in den Filter eindringen. Das Ventil kann beispielsweise beim Trocknen des Filters geschlossen werden, um ein Durchströmen des Filters über den Druckunterschied zwischen der ersten Kammer und der zweiten Kammer mit warmer Luft zu ermöglichen.

Dabei ist zweckmäßig, dass das Gehäuse der Druckkammer beheizbar ist.

Durch das Beheizen des Gehäuses der Druckkammer wird vorteilhaft das Kondensieren des Dampfes bei der Dampfsterilisation an der Gehäusewand verhindert bzw. die Kondensatmenge verringert.

Schließlich ist es zur Erfindung gehörig, dass die Vorrichtung Emitter zur Einbringung von Strahlungsenergie in die Filter aufweist.

Durch das Einbringen von Strahlungsenergie in die Filter können diese schneller getrocknet werden. Die Strahlung kann beispielsweise Mikrowellenstrahlung oder Infrarotstrahlung sein.

Die Aufgabe wird auch durch ein Verfahren zum Sterilisieren und Trocknen von Filtern, insbesondere medizinischen Filtern, gemäß Anspruch 11 gelöst. Das Verfahren besteht dabei aus den folgenden Schritten:
- Einlegen des Filters in die Halterung,
- Verschließen der Druckkammer der Vorrichtung,
- Zyklisches Evakuieren und Dampfeinlass in beide Kammern zur ersten Luftentfernung,
- Einleiten von Heißluft in die erste Kammer und Durchströmung des Filters für eine festgelegte Zeit zur Restluftentfernung,
- Beaufschlagen beider Kammern mit Heißdampf während einer festgelegten Zeit,
- Verdrängen des Heißdampfes über den Auslass in der ersten Kammer und / oder zweiten Kammer durch Einströmen von Warmluft in die erste Kammer und / oder zweite Kammer und Durchströmung des Filters bis zur vollständigen Trockenheit.

Die Aufgabe wird auch durch ein Verfahren mit den folgenden Schritten gelöst:
- Einlegen des Filters in die Halterung,
- Verschließen der Druckkammer der Vorrichtung,
- Einleiten von Heißdampf über den Einlass in die erste Kammer und/oder die zweite Kammer, um die in dem Filter befindliche Luft zu entfernen,
- Durchströmen des Filters mit Heißdampf während einer festgelegten Zeit,
- Entfernen des Heißdampfes über den Auslass in der zweiten Kammer durch Verdrängung mit Luft,
- Einleiten von warmer Luft in die erste Kammer und/oder die zweite Kammer und Durchströmen des Filters bis zur vollständigen Trocknung.

Die Aufgabe wird auch durch ein Verfahren mit den folgenden Schritten gelöst:
- Einlegen des Filters in die Halterung,
- Verschließen der Druckkammer der Vorrichtung,
- Zyklisches Evakuieren und Dampfeinlass in beide Kammern zur Luftentfernung,
- Beaufschlagen beider Kammern mit Heißdampf während einer festgelegten Zeit,
- Verdrängen des Heißdampfes über den Auslass in der ersten Kammer und / oder zweiten Kammer durch Einströmen von Warmluft in die erste Kammer und / oder zweiten Kammer,
- Einströmung von warmer Luft in die erste Kammer und / oder die zweite Kammer und Durchströmung des Filters bis zur vollständigen Trockenheit.

Die drei vorgenannten Verfahren werden jeweils mit einer erfindungsgemäßen Vorrichtung durchgeführt.

Bei einer bevorzugten Ausgestaltung des Verfahrens ist der Filter ein medizinischer Filter, insbesondere ein Dialysator, und der Blutraum des medizinischen Filters, insbesondere Dialysators, wird vor dem Sterilisationsprozess mit einer dampfdurchlässigen und mikrobenundurchlässigen Folie verschlossen. Die Hohlfasern des medizinischen Filters, insbesondere Dialysators, werden zunächst von dem Heißdampf durchströmt, dann mit diesem während einer festgelegten Zeit beaufschlagt und anschließend von der warmen Luft durchströmt.

Als Folie, die dampfdurchlässig und undurchlässig für Krankheitserreger, beispielsweise Mikroben ist, kann beispielsweise eine Folie aus Tyvek^{®} von DuPont verwendet werden. Durch die dampfdurchlässige und mikrobenundurchlässige Folie wird der Blutraum des medizinischen Filters, insbesondere Dialysators, pyrogendicht verschlossen. Besonders vorteilhaft müssen die Bluteinlassöffnung und die Blutauslassöffnung des medizinischen Filters, insbesondere Dialysators, nach dem Sterilisieren in der Vorrichtung nicht mehr steril verpackt werden.

Die dampfdurchlässige und mikrobenundurchlässige Folie kann dabei beispielsweise eine 0,2 Mikrometer dicke Membran aus pyrogendichter Folie sein.

Dabei ist bei Dialysatoren zweckmäßig, dass die Einlassöffnung und die Auslassöffnung für die Dialyseflüssigkeit während der Sterilisation verschlossen sind.

Vorteilhaft wird dadurch das Durchströmen des Blutraums des Dialysators begünstigt. Die Einlassöffnung und die Auslassöffnung für die Dialyseflüssigkeit können dabei beispielsweise mit einer Kappe verschlossen sein.

Schließlich ist es zur Erfindung gehörig, dass der Filter durch Energieeintrag oder durch Strahlung getrocknet wird.

Nachfolgend wird ein Ausführungsbeispiel der Erfindung anhand von Zeichnungen näher erläutert. Hierbei ist der Filter ein Dialysator.

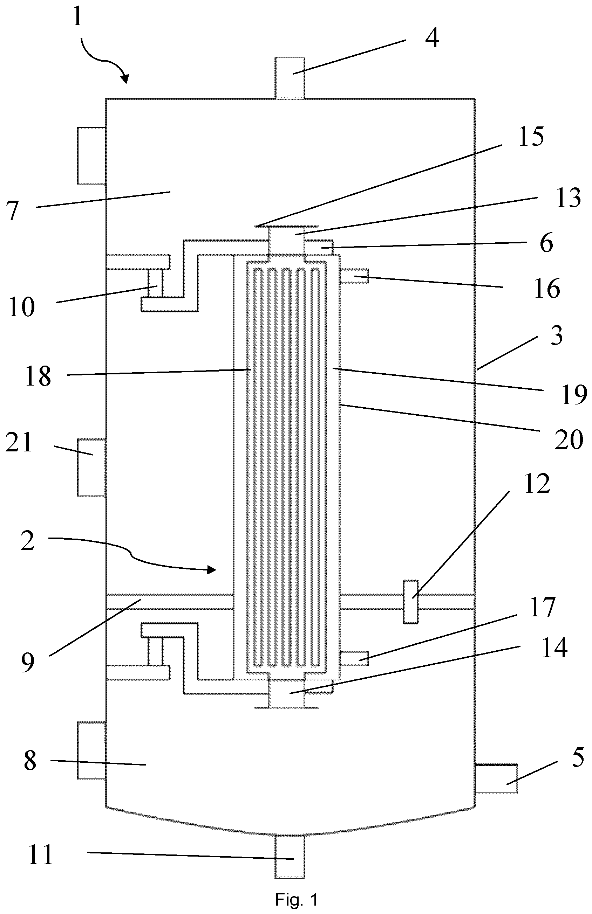

Es zeigt
- Fig. 1: einen Querschnitt einer erfindungsgemäßen Vorrichtung mit einem eingesetzten Dialysator.

Figur 1 zeigt eine schematische Darstellung einer Ausführungsform der erfindungsgemäßen Vorrichtung 1 mit eingesetztem Dialysator 2. Die Vorrichtung besteht aus einer zylindrischen Druckkammer 3 mit mindestens einem Einlass 4 und einem Auslass 5. Über den Einlass 4 kann Dampf oder warme Luft in die Druckkammer 3 eingeleitet werden. Mit Hilfe einer Vakuumpumpe kann auch Restluft aus den Kammern entfernt werden. Durch den Auslass 5 kann Dampf oder warme Luft aus der Druckkammer 3 ausgeleitet werden. Die Druckkammer 3 wird durch eine verstellbare Trennwand 9 in eine erste Kammer 7 und eine zweite Kammer 8 unterteilt, die vertikal übereinander angeordnet sind. Aufgrund der Verstellbarkeit der Trennwand 9 kann die Größe der ersten Kammer 7 und der zweiten Kammer 8 variiert werden. Die erste Kammer 7 und die zweite Kammer 8 sind durch ein Ventil 12 in der Trennwand 9 verbunden. Die Druckkammer 3 lässt sich durch eine Schließvorrichtung 21 öffnen und schließen. Am Boden der Vorrichtung 1 befindet sich ein Ablauf für Kondenswasser 11.

Innerhalb der Druckkammer befindet sich eine Halterung 6 zum Einsetzen des zu sterilisierenden Dialysators 2. Die Halterung besitzt ein Federelement 10 zum Aufbringen einer Kraft auf den Dialysator 2, um die Ausdehnung des Dialysatorgehäuses 20 während der Dampfsterilisation und anschließender Trocknung zu begrenzen. In der Mitte der Halterung 6 werden die Bluteinlassöffnung 13 bzw. die Blutauslassöffnung 14 des Blutraums des Dialysators 2 aufgenommen. Der Blutraum des Dialysators 2 wird durch Hohlfasern 18 gebildet. Zwischen der Einlassöffnung 16 und der Auslassöffnung 17 für Dialyseflüssigkeit befindet sich der Dialyseraum 19 des Dialysators 2. Die Bluteinlassöffnung 13 bzw. die Blutauslassöffnung 14 des Blutraums des Dialysators sind durch eine dampfdurchlässige und mikrobenundurchlässigen Folie 15 verschlossen. Durch die dampfdurchlässige und mikrobenundurchlässige Folie 15 können Dampf und Luft, jedoch keine gesundheitsgefährdenden Erreger in den Blutraum des Dialysators eindringen.

Um den Dialysator 2 zu sterilisieren, wird zunächst der Blutraum des Dialysators 2 mit einer dampfdurchlässigen und mikrobenundurchlässigen Folie 15, beispielsweise Tyvek^{®} von DuPont, verschlossen. Dazu wird jeweils eine dampfdurchlässige und mikrobenundurchlässige Folie 15 über die Bluteinlassöffnung 13 und Blutauslassöffnung 14, beispielsweise durch Thermoschweißen, angebracht. Im nächsten Schritt wird der Dialysator 2 in die Halterung 6 eingelegt und die Druckkammer 3 der Vorrichtung 1 geschlossen. Anschließend wird Dampf über den Einlass 4 in die erste Kammer 7 eingeleitet. Durch ein Druckgefälle zwischen der erstem Kammer 7 und der zweiten Kammer 8 werden die Hohlfasern des Dialysators 2 mit Dampf durchströmt und die darin befindliche Luft entfernt. Anschließend wird der Dialysator 2 mit Heißdampf während einer festgelegten Zeit, beispielsweise 15 min, durchströmt und dadurch sterilisiert. Der Heißdampf wird über den Auslass 5 in der zweiten Kammer 8 aus der Druckkammer 3 ausgeleitet. Zum Trocknen des Dialysators wird warme Luft über den Einlass 4 in die erste Kammer 7 eingeleitet. Durch ein Druckgefälle zwischen der ersten Kammer 7 und der zweiten Kammer 8 werden die Hohlfasern des Dialysators 2 mit der warmen Luft durchströmt, wobei die Feuchtigkeit von der warmen Luft aufgenommen wird. Die warme Luft wird über den Auslass 5 in der zweiten Kammer 8 aus der Druckkammer 3 entfernt.

## Patentansprüche

1. Vorrichtung (1) zum Sterilisieren und Trocknen von medizinischen Filtern wie Dialysatoren, wobei die Vorrichtung (1) eine Druckkammer (3) mit mindestens einem Einlass (4) und einem Auslass (5) und eine Halterung (6) für mindestens einen Filter (2) aufweist, **dadurch gekennzeichnet, dass** die Druckkammer in eine erste Kammer (7) und eine zweite Kammer (8) unterteilt ist, wobei der Filter (2) derart in die Halterung (5) einlegbar ist, dass die Einlassöffnung(en) (13, 16) des Filters (2) in der ersten Kammer (7) und die Auslassöffnung(en) (14, 17) des Filters (2) in der zweiten Kammer (8) angeordnet sind.

2. Vorrichtung (1) gemäß Anspruch 1, **dadurch gekennzeichnet, dass** die Druckkammer durch eine Trennwand (9) in eine erste Kammer (7) und eine zweite Kammer (8) unterteilt ist.

3. Vorrichtung gemäß Anspruch 2, **dadurch gekennzeichnet, dass** die Position der Trennwand (9) verstellbar ist.

4. Vorrichtung (1) gemäß einem die vorangegangenen Ansprüche, **dadurch gekennzeichnet, dass** die erste Kammer (7) größer als die zweite Kammer (8) ist.

5. Vorrichtung (1) gemäß einem der vorangegangenen Ansprüche, **dadurch gekennzeichnet, dass** im Bereich der Halterung (6) Mittel zum Aufbringen einer Kraft auf einen in der Halterung befindlichen Filter (2) vorgesehen sind.

6. Vorrichtung (1) gemäß Anspruch 5, **dadurch gekennzeichnet, dass zum** Aufbringen der Kraft auf einen in der Halterung (6) befindlichen Filter ein Federelement (10) oder ein Druckzylinder vorgesehen ist.

7. Vorrichtung (1) gemäß einem der vorangegangenen Ansprüche, **dadurch gekennzeichnet, dass** die Vorrichtung (1) im Boden der ersten Kammer (7) und der zweiten Kammer (8) jeweils einen Ablauf für Kondenswasser (11) aufweist.

8. Vorrichtung (1) gemäß einem der vorangegangenen Ansprüche, **dadurch gekennzeichnet, dass** die erste Kammer (7) und die zweite Kammer (8) durch ein Ventil (12) miteinander verbunden sind.

9. Vorrichtung (1) gemäß einem der vorangegangenen Ansprüche, **dadurch gekennzeichnet, dass** das Gehäuse der Druckkammer (3) beheizbar ist.

10. Vorrichtung (1) gemäß einem der vorangegangenen Ansprüche, **dadurch gekennzeichnet, dass** die Vorrichtung (1) Emitter zur Einbringung von Strahlungsenergie in die Filter aufweist.

11. Verfahren zum Sterilisieren und Trocknen von Filtern (2), insbesondere medizinischen Filtern wie Dialysatoren, mit einer Vorrichtung (1) gemäß einem der vorangegangenen Ansprüche, welches die folgenden Schritte umfasst:
• Einlegen des Filters (2) in die Halterung (6),
• Verschließen der Druckkammer (3) der Vorrichtung (1),
• Zyklisches Evakuieren und Dampfeinlass in beide Kammern zur ersten Luftentfernung,
• Einleiten von Heißluft in die erste Kammer (7) und Durchströmung des Filters (2) für eine festgelegte Zeit zur Restluftentfernung,
• Beaufschlagen beider Kammern mit Heißdampf während einer festgelegten Zeit,
• Verdrängen des Heißdampfes über den Auslass (5) in der ersten Kammer (7) und / oder zweiten Kammer (8) durch Einströmen von Warmluft in die erste Kammer (7) und / oder zweite Kammer (8) und Durchströmung des Filters (2) bis zur vollständigen Trockenheit.

12. Verfahren gemäß Anspruch 11, welches die folgenden Schritte umfasst:
• Einlegen des Filters (2) in die Halterung (6)
• Verschließen der Druckkammer (3) der Vorrichtung (1),
• Einleiten von Heißdampf über den Einlass (4) in die erste Kammer (7) und/oder die zweite Kammer (8), um die in dem Filter (2) befindliche Luft zu entfernen,
• Durchströmen des Filters (2) mit Heißdampf während einer festgelegten Zeit,
• Entfernen des Heißdampfes über den Auslass (5) in der zweiten Kammer (8) durch Verdrängung mit Luft,
• Einleiten von warmer Luft in die erste Kammer (7) und/oder die zweite Kammer (8) und Durchströmen des Filters bis zur vollständigen Trocknung.

13. Verfahren gemäß Anspruch 11 oder Anspruch 12, welches die folgenden Schritte umfasst:
• Einlegen des Filters (2) in die Halterung (6),
• Verschließen der Druckkammer (3) der Vorrichtung (1),
• Zyklisches Evakuieren und Dampfeinlass in beide Kammern zur Luftentfernung,
• Beaufschlagen beider Kammern mit Heißdampf während einer festgelegten Zeit,
• Verdrängen des Heißdampfes über den Auslass (5) in der ersten Kammer (7) und / oder zweiten Kammer (8) durch Einströmen von Warmluft in die erste Kammer (7) und / oder zweiten Kammer (8),
• Einströmung von warmer Luft in die erste Kammer (7) und / oder die zweite Kammer (8) und Durchströmung des Filters (2) bis zur vollständigen Trockenheit.

14. Verfahren gemäß einem der Ansprüche 11 bis 13, **dadurch gekennzeichnet, dass** der Filter (2) ein medizinischer Filter, insbesondere ein Dialysator, ist und der Blutraum des medizinischen Filters, insbesondere Dialysators, vor dem Einleiten von Heißdampf mit einer dampfdurchlässigen und mikrobenundurchlässigen Folie verschlossen wird.

15. Verfahren gemäß einem der Ansprüche 11 bis 14, **dadurch gekennzeichnet, dass** der Filter (2) durch Energieeintrag oder durch Strahlung getrocknet wird.

## Claims

1. Device (1) for sterilizing and drying medical filters such as dialyzers, wherein the device (1) comprises a pressure chamber (3) with at least one inlet (4) and one outlet (5) and a holder (6) for at least one filter (2), **characterized in that** the pressure chamber is divided into a first chamber (7) and a second chamber (8), wherein the filter (2) is insertable into the holder (5) in such a way that the inlet opening(s) (13, 16) of the filter (2) are arranged in the first chamber (7) and the outlet opening(s) (14, 17) of the filter (2) are arranged in the second chamber (8).

2. Device (1) according to claim 1, **characterized in that** the pressure chamber is divided into a first chamber (7) and a second chamber (8) by a partition (9).

3. Device according to claim 2, **characterized in that** the position of the partition (9) is adjustable.

4. Device (1) according to one of the preceding claims, **characterized in that** the first chamber (7) is larger than the second chamber (8).

5. Device (1) according to one of the preceding claims, **characterized in that** means for applying a force to a filter (2) located in the holder are provided in the region of the holder (6).

6. Device (1) according to claim 5, **characterized in that** a spring element (10) or a pressure cylinder is provided for applying the force to a filter located in the holder (6).

7. Device (1) according to one of the preceding claims, **characterized in that** the device (1) comprises a drain for condensed water (11) in the bottom of the first chamber (7) and the second chamber (8), respectively.

8. Device (1) according to one of the preceding claims, **characterized in that** the first chamber (7) and the second chamber (8) are interconnected by a valve (12).

9. Device (1) according to one of the preceding claims, **characterized in that** the housing of the pressure chamber (3) is heatable.

10. Device (1) according to one of the preceding claims, **characterized in that** the device (1) comprises emitters for introducing radiant energy into the filters.

11. Method for sterilizing and drying filters (2), in particular medical filters such as dialyzers, with an apparatus (1) according to one of the preceding claims, comprising the following steps:
• inserting the filter (2) into the holder (6),
• closing the pressure chamber (3) of the device (1),
• cyclic evacuation and steam inlet into both chambers for initial air removal,
• introducing hot air into the first chamber (7) and flowing through the filter (2) for a set time to remove residual air,
• pressurizing both chambers with superheated steam for a specified time,
• displacing the superheated steam via the outlet (5) in the first chamber (7) and / or second chamber (8) by flowing warm air into the first chamber (7) and / or second chamber (8) and flowing through the filter (2) until it is completely dry.

12. Method of claim 11, comprising the steps of:
• inserting the filter (2) into the holder (6)
• closing the pressure chamber (3) of the device (1),
• introducing superheated steam via the inlet (4) into the first chamber (7) and/or the second chamber (8) to remove the air present in the filter (2),
• flowing superheated steam through the filter (2) for a specified time,
• removing the superheated steam via the outlet (5) in the second chamber (8) by displacement with air,
• introducing warm air into the first chamber (7) and/or the second chamber (8) and flowing through the filter until it is completely dry.

13. Method according to claim 11 or claim 12, comprising the following steps:
• inserting the filter (2) into the holder (6),
• closing the pressure chamber (3) of the device (1),
• cyclic evacuation and steam inlet into both chambers for air removal,
• pressurizing both chambers with superheated steam for a specified time,
• displacing the superheated steam via the outlet (5) in the first chamber (7) and/or second chamber (8) by flowing warm air into the first chamber (7) and/or second chamber (8),
• inflow of warm air into the first chamber (7) and / or the second chamber (8) and flowing through the filter (2) until it is completely dry.

14. Method according to one of claims 11 to 13, **characterized in that** the filter (2) is a medical filter, in particular a dialyzer, and the blood space of the medical filter, in particular dialyzer, is sealed with a vapor-permeable and microbe-impermeable film before the introduction of superheated steam.

15. Method according to one of claims 11 to 14, **characterized in that** the filter (2) is dried by energy input or by radiation.

## Revendications

1. Dispositif (1) pour stériliser et sécher des filtres médicaux tels que des dialyseurs, le dispositif (1) présentant une chambre de pression (3) avec au moins une entrée (4) et une sortie (5) et un support (6) pour au moins un filtre (2), **caractérisé en ce que** la chambre de pression (3) est divisée en une première chambre (7) et une deuxième chambre (8), le filtre (2) pouvant être inséré dans le support (6) de telle sorte que la ou les ouvertures d'entrée (13, 16) du filtre (2) sont disposées dans la première chambre (7) et la ou les ouvertures de sortie (14, 17) du filtre (2) sont disposés dans la deuxième chambre (8).

2. Dispositif (1) selon la revendication 1, **caractérisé en ce que** la chambre de pression est divisée en une première chambre (7) et une deuxième chambre (8) par une cloison (9).

3. Dispositif selon la revendication 2, **caractérisé en ce que** la position de la cloison (9) est réglable.

4. Dispositif (1) selon l'une des revendications précédentes, **caractérisé en ce que** la première chambre (7) est plus grande que la deuxième chambre (8).

5. Dispositif (1) selon l'une des revendications précédentes, **caractérisé en ce que** des moyens pour appliquer une force sur un filtre (2) se trouvant dans le support sont prévus au niveau du support (6).

6. Dispositif (1) selon la revendication 5, **caractérisé en ce que** pour appliquer la force sur un filtre se trouvant dans le support (6), il est prévu un élément à ressort (10) ou un cylindre de pression.

7. Dispositif (1) selon l'une des revendications précédentes, **caractérisé en ce que** le dispositif (1) est muni dans le fond de la première chambre (7) et dans le fond de la deuxième chambre (8) d'une évacuation pour l'eau de condensation (11).

8. Dispositif (1) selon l'une des revendications précédentes, **caractérisé en ce que** la première chambre (7) et la deuxième chambre (8) sont reliées entre elles par une valve (12).

9. Dispositif (1) selon l'une des revendications précédentes, **caractérisé en ce que** le boîtier de la chambre de pression (3) peut être chauffé.

10. Dispositif (1) selon l'une des revendications précédentes, **caractérisé en ce que** le dispositif (1) est muni d'émetteurs pour introduire de l'énergie de rayonnement dans les filtres.

11. Procédé de stérilisation et de séchage de filtres (2), notamment de filtres médicaux tels que des dialyseurs, au moyen d'un dispositif (1) selon l'une des revendications précédentes, procédé comprenant les étapes suivantes :
• Mise en place du filtre (2) dans le support (6),
• Fermeture de la chambre de pression (3) du dispositif (1),
• Mise sous vide cyclique et admission de vapeur dans les deux chambres pour une première élimination de l'air,
• Introduction d'air chaud dans la première chambre (7) et passage dans le filtre (2) pendant une durée déterminée pour l'élimination de l'air résiduel,
• Introduction de vapeur surchauffée dans les deux chambres pendant une durée déterminée,
• Refoulement de la vapeur chaude par la sortie (5) dans la première chambre (7) et/ou la deuxième chambre (8) en faisant entrer de l'air chaud dans la première chambre (7) et/ou la deuxième chambre (8) et en le faisant passer dans le filtre (2) jusqu'au séchage complet.

12. Procédé selon la revendication 11, comprenant les étapes suivantes :
• Mise en place du filtre (2) dans le support (6) ;
• Fermeture de la chambre de pression (3) du dispositif (1),
• Introduction de vapeur surchauffée par l'entrée (4) dans la première chambre (7) et/ou la deuxième chambre (8) afin d'éliminer l'air présent dans le filtre (2),
• Passage de la vapeur chaude dans le filtre (2) pendant une durée déterminée,
• Élimination de la vapeur surchauffée par la sortie (5) dans la deuxième chambre (8) par refoulement avec de l'air,
• Introduction d'air chaud dans la première chambre (7) et/ou la deuxième chambre (8) et passage à travers le filtre jusqu'au séchage complet.

13. Procédé selon la revendication 11 ou la revendication 12, comprenant les étapes suivantes :
• Mise en place du filtre (2) dans le support (6),
• Fermeture de la chambre de pression (3) du dispositif (1),
• Mise sous vide cyclique et admission de vapeur dans les deux chambres pour éliminer l'air,
• Introduction de vapeur surchauffée dans les deux chambres pendant une durée déterminée,
• Refoulement de la vapeur surchauffée par la sortie (5) dans la première chambre (7) et/ou la deuxième chambre (8) en faisant entrer de d'air chaud dans la première chambre (7) et/ou la deuxième chambre (8),
• Introduction d'air chaud dans la première chambre (7) et/ou la deuxième chambre (8) et passage dans le filtre (2) jusqu'au séchage complet.

14. Procédé selon l'une des revendications 11 à 13, **caractérisé en ce que** le filtre (2) est un filtre médical, notamment un dialyseur, et **en ce que** l'espace sanguin du filtre médical, notamment du dialyseur, est fermé par un film perméable à la vapeur et imperméable aux microbes avant l'introduction de la vapeur surchauffée.

15. Procédé selon l'une des revendications 11 à 14, **caractérisé en ce que** le filtre (2) est séché par apport d'énergie ou par rayonnement.
